(19) Europäisches Patentamt
European Patent Office
Office européen des brevets

(11) **EP 2 307 062 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**24.11.2021 Patentblatt 2021/47**

(45) Hinweis auf die Patenterteilung:
**05.03.2014 Patentblatt 2014/10**

(21) Anmeldenummer: **09780136.9**

(22) Anmeldetag: **03.07.2009**

(51) Int Cl.:
*A61L 15/22* (2006.01)      *A61L 15/60* (2006.01)
*C08J 3/12* (2006.01)       *C08J 3/24* (2006.01)
*C08J 11/04* (2006.01)      *C08J 11/06* (2006.01)
*C08L 101/14* (2006.01)

(86) Internationale Anmeldenummer:
**PCT/EP2009/058416**

(87) Internationale Veröffentlichungsnummer:
**WO 2010/006937 (21.01.2010 Gazette 2010/03)**

(54) **VERFAHREN ZUR HERSTELLUNG WASSERABSORBIERENDER POLYMERPARTIKEL**

METHOD FOR PRODUCING WATER-ABSORBING POLYMER PARTICLES

PROCÉDÉ POUR PRODUIRE DES PARTICULES POLYMÈRES HYDROPHILES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **15.07.2008 EP 08160430**

(43) Veröffentlichungstag der Anmeldung:
**13.04.2011 Patentblatt 2011/15**

(73) Patentinhaber: **BASF SE**
**67056 Ludwigshafen am Rhein (DE)**

(72) Erfinder:
• **FUNK, Rüdiger**
**65527 Niedernhausen (DE)**
• **WEISMANTEL, Matthias**
**63637 Jossgrund (DE)**
• **BERTHA, Sylvia**
**67227 Frankenthal (DE)**

(74) Vertreter: **BASF IP Association**
**BASF SE**
**G-FLP-C006**
**67056 Ludwigshafen (DE)**

(56) Entgegenhaltungen:
**EP-A- 0 695 763        EP-A1- 1 690 887**
**WO-A-2004/018006    WO-A1-2004/018006**
**WO-A1-2009/153196   WO-A2-2007/074167**
**US-A- 5 486 569**

Bemerkungen:
Die Akte enthält technische Angaben, die nach dem Eingang der Anmeldung eingereicht wurden und die nicht in dieser Patentschrift enthalten sind.

EP 2 307 062 B2

**Beschreibung**

Die vorliegende Erfindung betrifft ein Verfahren zur Herstellung wasserabsorbierender Polymerpartikel, umfassend Polymerisation, Trocknung, Zerkleinerung, Klassierung und Rückführung des bei der Klassierung anfallenden Unterkorns, wobei das rückgeführte Unterkorn mit einem Reduktionsmittel und/oder anorganischen Partikeln beschichtet ist.

Wasserabsorbierende Polymere werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymere werden auch als Superabsorber bezeichnet und bestehen aus hydrophilen Polymeren, die so stark vernetzt sind, dass sie nicht mehr löslich sind.

Die Herstellung der wasserabsorbierenden Polymere wird beispielsweise in der Monographie "Modern Superabsorbent Polymer Technology", F.L. Buchholz und A.T. Graham, Wiley-VCH, 1998, Seiten 71 bis 103, und in Ullmann's Encyclopedia of Industrial Chemistry, 6. Auflage, Band 35, Seiten 73 bis 93, beschrieben.

Die Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm$^2$ (AUL0.3psi) durchläuft ein Maximum.

Zur Verbesserung der Anwendungseigenschaften, wie beispielsweise Permeabilität des gequollenen Gelbetts (SFC) in der Windel und Absorption unter einem Druck von 49.2 g/cm$^2$ (AUL0.7psi), werden wasserabsorbierende Polymerpartikel im allgemeinen oberflächennachvernetzt. Dadurch steigt der Vernetzungsgrad der Partikeloberfläche, wodurch die Absorption unter einem Druck von 49,2 g/cm$^2$ (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet und thermisch oberflächennachvernetzt.

Die wasserabsorbierenden Polymere gelangen als pulverförmiges, körniges Produkt bevorzugt im Hygienesektor zum Einsatz. Hier werden beispielsweise Teilchengrößen zwischen 150 und 850 μm eingesetzt und das partikuläre Polymermaterial wird bereits beim Herstellungsprozess auf diese Korngrößen klassiert. Hierbei werden kontinuierlich arbeitende Siebmaschinen mit mindestens zwei Sieben eingesetzt, wobei Siebe mit den Maschenweiten von 150 und 850 μm verwendet werden. Partikel mit einer Korngröße von bis zu 150 μm fallen dabei durch beide Siebe und werden am Boden der Siebmaschine als Unterkorn gesammelt, ausgeschleust und rückgeführt. Partikel mit einer Korngröße von größer 850 μm verbleiben als Überkorn auf dem obersten Sieb und werden ausgeschleust, erneut gemahlen und rückgeführt. Die Produktfraktion mit einer Korngröße von größer 150 bis 850 μm wird als Mittelkorn zwischen den beiden Sieben der Siebmaschine entnommen.

Das bei der Klassierung anfallende Unter- und Überkorn wird üblicherweise in den Produktionsprozess zurückgeführt. Die Rückführung des Unterkorns wird beispielsweise in EP 0 463 388 A1, EP 0 496 594 A2, EP 0 785 224 A2, EP 1 878 761 A1 und US 5,064,582 beschrieben.

EP 0 463 388 A1 beschreibt, dass durch Zusatz von wenig Unterkorn zu Polymergelen mit niedrigem Feststoffgehalt pumpbare Polymergele mit hohem Feststoffgehalt erhalten werden können.

EP 0 496 594 A2 lehrt die Rückführung des Unterkorns in den Polymerisationsreaktor.

EP 0 785 224 A2 beschreibt die Rückführung des Unterkorns in das bei der Polymerisation entstandene Polymergel, wobei Tenside zugesetzt werden.

EP 1 878 761 A1 offenbart ein Verfahren zur Rückführung eines mit wasserlöslichen polyvalenten Metallsalzen beschichteten Unterkoms. Das Unterkorn kann beispielsweise mittels eines Kneters in das Polymergel eingemischt werden.

US 5,064,582 offenbart ein Verfahren zur Rückführung von Unterkorn, wobei das Unterkorn vor der Rückführung mit Wasser vermischt wird.

Gemäß WO 2004/018006 A1 kann die Menge an rückzuführendem Unterkorn deutlich reduziert werden. Bei dem dort beschriebenen Verfahren wird auf die Abtrennung des Unterkorns vor der Oberflächennachvernetzung verzichtet. Statt dessen wird das Unterkorn durch Zusatz von Tonmineralen bei der Oberflächennachvernetzung zu 80 bis 85% gebunden.

Aufgabe der vorliegenden Erfindung war die Bereitstellung eines verbesserten Verfahrens zur Rückführung des bei der Herstellung wasserabsorbierender Polymerpartikel anfallenden Unterkorns.

Gelöst wurde die Aufgabe durch ein Verfahren zur Herstellung wasserabsorbierender Polymerpartikel durch Polymerisation einer Monomerlösung oder -suspension, enthaltend

a) mindestens ein ethylenisch ungesättigtes, säuregruppentragendes Monomer, das zumindest teilweise neutralisiert sein kann,
b) mindestens einen Vernetzer,
c) mindestens einen Initiator,

d) optional ein oder mehrere mit den unter a) genannten Monomeren copolymerisierbare ethylenisch ungesättigte Monomere und

e) optional ein oder mehrere wasserlösliche Polymere,

umfassend die Schritte

i) Polymerisation der Monomerlösung oder -suspension zu einem Polymergel,
ii) Trocknung des Polymergels,
iii) Zerkleinerung des getrockneten Polymergels und
iv) Klassierung, wobei Unterkorn abgetrennt wird,

wobei das abgetrennte Unterkorn zumindest teilweise vor Schritt ii) rückgeführt wird, dadurch gekennzeichnet, dass die wasserabsorbierenden Polymerpartikel vor Schritt iv) oberflächennachvernetzt werden, dass nach Schritt iii) und vor der Oberflächennachvernetzung zusätzlich klassiert und Unterkorn abgetrennt wird und dass zumindest eine Teilmenge des rückgeführten Unterkorns mit mindestens einem Reduktionsmittel und/oder anorganischen Partikeln beschichtet ist, wobei sie vor Schritt iv) und nach der Oberflächennachvernetzung mit einem Reduktionsmittel und/oder anorganischen Partikeln beschichtet wird.

Das das vor der Oberflächennachvernetzung abgetrennte Unterkom als auch das nach der Oberflächennachvernetzung abgetrennte Unterkom werden vorzugsweise in das Verfahren rückgeführt, besonders bevorzugt als Mischung. Das Mischungsverhältnis unterliegt keiner Beschränkung, es sollte aber ein möglichst hoher Anteil an nicht oberflächennachvernetztem Unterkom angestrebt werden.

Besonders vorteilhaft ist die Rückführung des Unterkorn nach der Polymerisation i), d.h. in einen separaten Apparat zwischen Polymerisationsreaktor und Trockner. Geeignete Apparate hierfür sind beispielsweise Kneter und Extruder.

Die rückgeführte Menge an mit Reduktionsmittel und/oder anorganischen Partikeln beschichteten Unterkorn beträgt vorzugsweise 1 bis 20 Gew.-%, besonders bevorzugt 2 bis 15 Gew.-%, ganz besonders bevorzugt 5 bis 10 gew.-%, jeweils bezogen auf den Feststoffgehalt der Monomerlösung. Der Feststoffgehalt ist die Summe aller nach der Polymerisation nichtflüchtigen Bestandteile. Dies sind das Monomer a), der Vernetzer b), der Initiator c), das Monomer d) und das Polymer e).

Die Menge an Reduktionsmittel beträgt vorzugsweise 0,01 bis 5 Gew.-%, besonders bevorzugt 0,05 bis 2 Gew.-%, ganz besonders bevorzugt 0,1 bis 1 Gew.-%, jeweils bezogen auf das beschichtete Unterkorn.

Die Reduktionsmittel unterliegen keiner Beschränkung. Geeignete Reduktionsmittel sind beispielsweise Natriumsulfit, Natriumhydrogensulfit (Natriumbisulfit), Natriumdithionit, Sulfinsäuren und deren Salze, Ascorbinsäure, Natriumhypophosphit, Natriumphosphit, sowie Phosphinsäuren und deren Salze. Vorzugsweise werden aber Salze der unterphosphorigen Säure, beispielsweise Natriumhypophosphit, und Salze von Sulfinsäuren verwendet, beispielsweise das Dinatriumsalz der 2-Hydroxy-2-sulfinatoessigsäure.

Die Reduktionsmittel werden üblicherweise als Lösung in einem geeigneten Lösungsmittel, vorzugsweise Wasser, eingesetzt.

Die Menge an anorganischen Partikeln beträgt vorzugsweise 0,05 bis 5 Gew.-%, besonders bevorzugt 0,1 bis 2 Gew.-%, ganz besonders bevorzugt 0,2 bis 1 Gew.-%, jeweils bezogen auf das beschichtete Unterkorn.

Geeignete anorganische Partikel sind beispielsweise Aluminiumsulfat, Magnesiumsulfat, Tonminerale, Kalziumsulfat, Magnesiumkarbonat, Kaliumaluminiumsulfat (Kaliumalaun), Aluminiumnitrat, Aluminiumchlorid, Natriumaluminiumsulfat (Natriumalaun), Magnesiumoxid, Aluminiumoxid, Diatomeenerde, Titandioxid, Sand und Zeolithe. Vorzugsweise werden aber wasserunlösliche anorganische Partikel verwendet, beispielsweise pyrogene Kieselsäure und wasserunlösliche Metallphosphate, wie Kalziumphosphat. Wasserunlöslich bedeutet hierbei eine Löslichkeit in Wasser bei 23°C von weniger als 1 g/100 g Wasser, vorzugsweise von weniger als 0,5 g/100 g Wasser, besonders bevorzugt von weniger als 0,1 g/100 g Wasser, ganz besonders bevorzugt von weniger als 0,05 g/100 g Wasser.

Die anorganischen Partikel weisen eine mittlere Partikelgröße von vorzugsweise weniger als 400 $\mu$m, besonders bevorzugt weniger als 100 $\mu$m, ganz besonders bevorzugt weniger als 50 $\mu$m, auf. Wasserunlösliche anorganische Partikel können auch als wässrige Dispersion eingesetzt werden.

Kristalline anorganische Feststoffe weisen vorzugsweise eine Partikelgröße von größer 10 $\mu$m auf. Die zum Beschichten des Unterkoms einsetzbaren Apparate unterliegen keiner Beschränkung. Geeignete Mischer mit bewegten Mischwerkzeugen sind beispielsweise Schneckenmischer, Scheibenmischer und Schaufelmischer.

Mischer mit rotierenden Mischwerkzeugen werden gemäß der Lage der Rotationsachse zur Produktstromrichtung in Vertikalmischer und Horizontalmischer unterteilt. Vorteilhaft werden Horizontalmischer verwendet. Besonders bevorzugt werden kontinuierliche Horizontalmischer (Durchlaufmischer) verwendet.

Die Verweilzeit im Horizontalmischer beträgt vorzugsweise von 1 bis 180 Minuten, besonders bevorzugt von 2 bis 60 Minuten, ganz besonders bevorzugt von 5 bis 20 Minuten.

Die Umfangsgeschwindigkeit der Mischwerkzeuge im Horizontalmischer beträgt vorzugsweise von 0,1 bis 10 m/s, besonders bevorzugt von 0,5 bis 5 m/s, ganz besonders bevorzugt von 0,75 bis 2,5 m/s.

Die wasserabsorbierenden Polymerpartikel werden im Horizontalmischer mit einer Geschwindigkeit bewegt, die einer Froude-Zahl von vorzugsweise 0,01 bis 6, besonders bevorzugt 0,05 bis 3, ganz besonders bevorzugt 0,1 bis 0,7, entspricht.

Zur Beschichtung wird vorzugsweise eine thermisch isolierte und/oder begleitbeheizte Zweistoffdüse eingesetzt, die vorteilhaft unter der Produktbettoberfläche endet.

Im Folgenden wird die Herstellung der, üblicherweise wasserunlöslichen, wasserabsorbierenden Polymerpartikel näher erläutert.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1,
EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraalloxyethan, Methylenbismethacrylamid, 15-fach ethoxiliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3-bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-

%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 g/cm$^2$ (AUL0.3psi)durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; DE) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren für die Polymerisation i) sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Die Trocknung ii) des Polymergels wird dann vorzugsweise mit einem Bandtrockner durchgeführt bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA (European Disposables and Nonwovens Association) empfohlenen Testmethode Nr. WSP 230.2-05 "Moisture Content" bestimmt wird. Bei einer zu hohen Restfeuchte weist das getrocknete Polymergel eine zu niedrige Glasübergangstemperatur Tg auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach zerkleinert und klassiert. Zur Zerkleinerung iii) werden üblicherweise ein- oder mehrstufige Walzenstühle, bevorzugt zwei- oder dreistufige Walzenstühle, Stiftmühlen, Hammermühlen oder Schwingmühlen, eingesetzt. Zur Klassierung iv) werden üblicherweise Taumelsiebmaschinen verwendet.

Die mittlere Partikelgröße der als Produktfraktion abgetrennten Polymerpartikel beträgt vorzugsweise mindestens 200 μm, besonders bevorzugt von 250 bis 600 μm, ganz besonders von 300 bis 500 μm. Die mittlere Partikelgröße der Produktfraktion kann mittels der von der EDANA (European Disposables and Nonwovens Association) empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 μm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Die geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Der Anteil an Partikeln mit einer Partikelgröße von höchstens 850 μm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Zerkleinerung iii) des getrockneten Polymergels rückgeführt.

Die Polymerpartikel werden zur weiteren Verbesserung der Eigenschaften oberflächennachvernetzt. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder poly-funktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidy-lether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyloxazolidin-2-on, Oxazolidin-2-on und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Laktat, möglich. Aluminiumsulfat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird.

Im Anschluss an das Aufsprühen werden die mit Oberflächennachvernetzer beschichteten Polymerpartikel thermisch getrocknet, wobei die Oberflächennachvemetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; DE), Vrieco-Nauta Continuous Mixer (Hosokawa Micron BV; Doetinchem; NL), Processall Mixmill Mixer (Processall Incorporated; Cincinnati; US) und Schugi Flexomix® (Hosokawa Micron BV; Doetinchem; NL). Es ist aber auch möglich die O-berflächennachvemetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandioi/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknem, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; DE), Hosokawa Bepex® Disc Dryer (Hosokawa Micron GmbH; Leingarten; DE) und Nara Paddle Dryer (NARA Machinery Europe; Frechen; DE). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel werden zur weiteren Verbesserung der Eigenschaften beschichtet und können nachbefeuchtet werden. Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Wird eine derartige Beschichtung oder Nachbefeuchtung durchgeführt, so werden die oberflächennachvernetzten Polymerpartikel vorteilhaft erst nach der Beschichtung bzw. Nachbefeuchtung erneut klassiert.

Die gemäß dem erfindungsgemäßen Verfahren hergestellten wasserabsorbierenden Polymerpartikel weisen einen Feuchtegehalt von vorzugsweise 0 bis 15 Gew.-%, besonders bevorzugt 0,2 bis 10 Gew.-%, ganz besonders bevorzugt 0,5 bis 8 Gew.-%, auf, wobei der Wassergehalt gemäß der von der EDANA (European Disposables and Nonwovens Association) empfohlenen Testmethode Nr. WSP 230.2-05 "Moisture Content" bestimmt wird.

Die gemäß dem erfindungsgemäßen Verfahren hergestellten wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA (European Disposables and Nonwovens Association) empfohlenen Testmethode Nr. WSP 241.2-05 "Centrifuge Retention Capacity" bestimmt.

Die gemäß dem erfindungsgemäßen Verfahren hergestellten wasserabsorbierenden Polymerpartikel weisen eine Absorption unter einem Druck von 49,2 $g/cm^2$ (AUL0.7psi) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Absorption unter einem Druck von 49,2 g/cm$^2$ (AUL0.7psi) der wasserabsorbierenden Polymerpartikel beträgt üblicherweise weniger als 35 g/g. Die Absorption unter einem Druck von 49,2 g/cm$^2$ (AUL0.7psi) wird analog der von der EDANA (European Disposables and Nonwovens Association) empfohlenen Testmethode Nr. WSP 242.2-05 "Absorption under Pressure" bestimmt, wobei statt eines Drucks von 21,0 g/cm$^2$ (AUL0.3psi) ein Druck von 49,2 g/cm$^2$ (AUL0.7psi) eingestellt wird.

Methoden:

Die Messungen sollten, wenn nicht anders angegeben, bei einer Umgebungstemperatur von 23 $\pm$ 2 °C und einer relativen Luftfeuchte von 50 $\pm$ 10 % durchgeführt werden. Die wasserabsorbierenden Polymerpartikel werden vor der Messung gut durchmischt.

Restmonomere (Residual Monomers)

Die Restmonomeren (Remos) werden gemäß der von der EDANA (European Disposables and Nonwovens Association) empfohlenen Testmethode Nr. WSP 210.2-05 "Residual Monomers" bestimmt.

Korngrößenverteilung (Particle Size Distribution)

Die Korngrößenverteilung (PSD) werden gemäß der von der EDANA (European Disposables and Nonwovens Association) empfohlenen Testmethode Nr. WSP 220.2-05 "Particle Size Distribution" bestimmt.

Zentrifugenretentionskapazität (Centrifuge Retention Capacity)

Die Zentrifugenretentionskapazität (CRC) wird gemäß der von der EDANA (European Disposables and Nonwovens Association) empfohlenen Testmethode Nr. WSP 241.2-05 "Centrifuge Retention Capacity" bestimmt.

Absorption unter einem Druck von 21,0 g/cm$^2$ (Absorption under Pressure)

Die Absorption unter einem Druck von 21,0 g/cm$^2$ (AUL0.3psi) wird gemäß der von der EDANA (European Disposables and Nonwovens Association) empfohlenen Testmethode Nr. WSP 242.2-05 "Absorption under Pressure" bestimmt.

Absorption unter einem Druck von 49,2 g/cm$^2$ (Absorption under Pressure)

Die Absorption unter einem Druck von 49,2 g/cm$^2$ (AUL0.7psi) wird analog der von der EDANA (European Disposables and Nonwovens Association) empfohlenen Testmethode Nr. WSP 242.2-05 "Absorption under Pressure" bestimmt, wobei statt eines Drucks von 21,0 g/cm$^2$ (AUL0.3psi) ein Druck von 49,2 g/cm$^2$ (AUL0.7psi) eingestellt wird.

Anquellgeschwindigkeit (Free Swell Rate)

Zur Bestimmung der Anquellgeschwindigkeit (FSR) werden 1,00 g (= $W_1$) der trockenen wasserabsorbierenden Polymerpartikel in ein 25 ml Becherglas eingewogen und gleichmäßig auf dessen Boden verteilt. Dann werden 20 ml einer 0,9 gew.-%igen Kochsalzlösung in ein zweites Becherglas dosiert. Der Inhalt dieses Glases wird dem ersten zügig hinzugefügt und eine Stoppuhr gestartet. Sobald der letzte Tropfen Salzlösung absorbiert wurde, was man am Verschwinden der Reflexion auf der Flüssigkeitsoberfläche erkennt, wird die Stoppuhr angehalten. Die genaue Flüssigkeitsmenge, die aus dem zweiten Becherglas ausgegossen und durch das Polymer im ersten Becherglas absorbiert wurde, wird durch Rückwägung des zweiten Becherglases genau bestimmt (= $W_2$). Die für die Absorption benötigte Zeitspanne, die mit der Stoppuhr gemessen wurde, wird als t bezeichnet.

Daraus errechnet sich die Anquellgeschwindigkeit (FSR) wie folgt:

$$\text{FSR [g/gs]} = W_2/(W_1 \text{x} t)$$

CIE-Farbzahl (L* a* b*)

Die Farbmessung wurde entsprechend dem CIELAB-Verfahren (Hunterlab, Band 8, Jahrgang 1996, Heft 7, Seiten 1 bis 4) durchgeführt. Dabei werden die Farben über die Koordinaten L*, a* und b* eines dreidimensionalen Systems beschrieben. Dabei gibt L* die Helligkeit an, wobei L* = 0 schwarz und L* = 100 weiß bedeutet. Die Werte für a* und b* geben die Position der Farbe auf den Farbachsen rot/grün bzw. gelb/blau an, wobei +a* für rot, -a* für grün, +b* für gelb und -b* für blau steht. Verwendet wurde ein Colorimeter: "Modell LabScan XE S/N LX17309" (HunterLab, Reston, US).

Es wurden folgende Geräteeinstellungen verwendet: Lichtquelle C; Beobachter 2°; Geometrie 45/0.

Die Farbmessung entspricht dem Dreibereichsverfahren nach DIN 5033-6.

Der Hunter 60-Wert (H60) ist ein Maß für den Weißgrad von Oberflächen und ist definiert als L*-3b*, d.h., der Wert ist umso niedriger, je dunkler und je gelbstichiger eine Farbe ist.

Die EDANA-Testmethoden sind beispielsweise erhältlich bei der European Disposables and Nonwovens Association, Avenue Eugène Plasky 157, B-1030 Brüssel, Belgien.

Beispiele:

Beispiel 1 (Herstellung des Grundpolymers) (Vergleichsbeispiel)

1028 g einer 37,3 gew.-%igen wässrigen Natriumacrylatlösung, 98 g Acrylsäure, 254 g Wasser und 0,59 g 3-fach ethoxiliertes Glyzerintriacrylat wurden in einen 2.000 ml Metallbecher eingewogen. Der Neutralisationsgrad betrug 75 mol-%. Der Metallbecher wurde mit Parafilm® verschlossen und mit 150l/h Stickstoff inertisiert. Während des Inertisierens wurde die Monomerlösung auf-0,5°C gekühlt. Anschließend wurden nacheinander 6,47 g einer 10 gew.-%igen wässrigen Lösung von Natriumpersulfat und 5,88 g einer 1 gew.-%igen wässrigen Lösung von Wasserstoffperoxid zugegeben.

Die Monomerlösung wurde mittels eines Trichters in eine Glasschale mit einem Durchmesser von 190 mm überführt. Die Glasschale wurde mit einer Kunststofffolie abgedeckt und ebenfalls mit 150l/h Stickstoff inertisiert. Zusätzlich wurde die Monomerlösung in der Glasschale mittels eines Magnetrührstabes gerührt. Anschließend wurden mittels einer Einwegspritze 5,88 g einer 1 gew.-%igen wässrigen Lösung von Brüggolite®FF6 (Dinatriumsalz der 2-Hydroxy-2-sulfinatoessigsäure) in die Monomerlösung dosiert. Nach dem Reaktionsstart wurde der Magnetrührer abgeschaltet.

Nach einer Reaktionszeit von 30 Minuten wurde das Polymergel entnommen und mit einem Extruder mit Lochplatte (6 mm Lochdurchmesser) zerkleinert, mit 17,6 g einer 1 gew.-%igen wässrigen Lösung von Natriumbisulfit besprüht und zweimal extrudiert.

Das Gel wurde eine Stunde bei 160°C im Umlufttrockenschrank getrocknet. Die Beladung der Bleche mit Polymergel betrug 0,59 g/cm². Anschließend wurde mit einem vierstufigen Walzenstuhl mit einer Spaltbreite von 5 mm, 1.000 $\mu$m, 600 $\mu$m und 400 $\mu$m zerkleinert und auf 150 bis 850 $\mu$m abgesiebt (Grundpolymer).

Die erhaltenen wasserabsorbierenden Polymerpartikel wurden analysiert. Die Ergebnisse sind in Tabelle 1 zusammengefasst.

Eine Teilmenge des Grundpolymers wurde mittels einer Rotormühle (Retsch® ZM200) auf eine Partikelgröße von weniger als 150 $\mu$m zerkleinert (Unterkorn).

Beispiel 2 (Vergleichsbeispiel)

1028 g einer 37,3 gew.-%igen wässrigen Natriumacrylatlösung, 98 g Acrylsäure, 254 g Wasser und 0,59 g 3-fach ethoxiliertes Glyzerintriacrylat wurden in einen 2.000 ml Metallbecher eingewogen. Der Neutralisationsgrad betrug 75 mol-%. Der Metallbecher wurde mit Parafilm® verschlossen und mit 150l/h Stickstoff inertisiert. Während des Inertisierens wurde die Monomerlösung auf-0,5°C gekühlt. Anschließend wurden nacheinander 6,47 g einer 10gew.-%igen wässrigen Lösung von Natriumpersulfat und 5,88 g einer 1 gew.-%igen wässrigen Lösung von Wasserstoffperoxid zugegeben.

Die Monomerlösung wurde mittels eines Trichters in eine Glasschale mit einem Durchmesser von 190 mm überführt. Die Glasschale wurde mit einer Kunststofffolie abgedeckt und ebenfalls mit 150l/h Stickstoff inertisiert. Zusätzlich wurde die Monomerlösung in der Glasschale mittels eines Magnetrührstabes gerührt. Anschließend wurden mittels einer Einwegspritze 5,88 g einer 1 gew.-%igen wässrigen Lösung von Brüggolite®FF6 (Dinatriumsalz der 2-Hydroxy-2-sulfinatoessigsäure) in die Monomerlösung dosiert. Nach dem Reaktionsstart wurde der Magnetrührer abgeschaltet.

Nach einer Reaktionszeit von 30 Minuten wurde das Polymergel entnommen und mit einem Extruder mit Lochplatte (6 mm Lochdurchmesser) zerkleinert, mit 17,6 g einer 1 gew.-%igen wässrigen Lösung von Natriumbisulfit besprüht und erneut extrudiert. Anschließend wurden insgesamt 84 g Unterkorn aus Beispiel 1 in zwei Portionen mittels eines 180 $\mu$m Siebes und eines Löffels aufgepudert und ein drittes Mal extrudiert.

Das Gel wurde eine Stunde bei 160°C im Umlufttrockenschrank getrocknet. Die Beladung der Bleche mit Polymergel betrug 0,59 g/cm². Anschließend wurde mit einem vierstufigen Walzenstuhl mit einer Spaltbreite von 5 mm,

1.000 $\mu$m, 600 $\mu$m und 400 $\mu$m zerkleinert und auf 150 bis 850 $\mu$m abgesiebt (Grundpolymer).

Die erhaltenen wasserabsorbierenden Polymerpartikel wurden analysiert. Die Ergebnisse sind in Tabelle 1 zusammengefasst.

Beispiel 3 (Vergleichsbeispiel)

Es wurde verfahren wie unter Beispiel 2. Das in das nach der Polymerisation erhaltene Polymergel eingearbeitete Unterkorn wurde vor der Rückführung mit Natriumhypophosphit beschichtet.

Die erhaltenen wasserabsorbierenden Polymerpartikel wurden analysiert. Die Ergebnisse sind in Tabelle 1 zusammengefasst.

Herstellung des mit Natriumhypophosphit beschichteten Unterkorns:

Dazu wurden 84 g Unterkorn aus Beispiel 1 in eine Küchenmaschine eingefüllt und gerührt. Auf das gerührte Unterkorn wurde mittels eines Zweistoffdüse 16,8 g einer 3 gew.-%igen wässrigen Natriumhypophosphitlösung aufgesprüht und 5 Minuten nachgerührt. Das beschichtete Unterkorn wurde auf vier Petrischalen (Durchmesser 13,5 cm; Höhe 2,5 cm) verteilt und 30 Minuten bei 150°C im Umlufttrockenschrank getrocknet. Anschließend wurde in einer Kaffeemühle deagglomeriert und auf kleiner 150 $\mu$m abgesiebt.

Beispiel 4 (Vergleichsbeispiel)

Es wurde verfahren wie unter Beispiel 2. Das in das nach der Polymerisation erhaltene Polymergel eingearbeitete Unterkorn wurde vorder Rückführung mit Brüggolite® FF7 (Brüggemann Chemicals; Heilbronn; DE) beschichtet. Brüggolit®FF7 ist ein Gemisch aus dem Dinatriumsalz der 2-Hydroxy-2-sulfinatoessigsäure, dem Dinatriumsalz der 2-Hydroxy-2-sulfonatoessigsäure und Natriumbisulfit.

Die erhaltenen wasserabsorbierenden Polymerpartikel wurden analysiert. Die Ergebnisse sind in Tabelle 1 zusammengefasst.

Herstellung des mit Brüggolite® FF7 beschichteten Unterkorns:

Dazu wurden 84 g Unterkorn aus Beispiel 1 in eine Küchenmaschine eingefüllt und gerührt. Auf das gerührte Unterkorn wurde mittels eines Zweistoffdüse 8,4 g einer 1 gew.-%igen wässrigen Lösung von Brüggolite® FF7 aufgesprüht und 5 Minuten nachgerührt. Das beschichteten Unterkorn wurde auf vier Petrischalen (Durchmesser 13,5 cm; Höhe 2,5 cm) verteilt und 30 Minuten bei 150°C im Umlufttrockenschrank getrocknet. Anschließend wurde in einer Kaffeemühle deagglomeriert und auf kleiner 150 $\mu$m abgesiebt.

Beispiel 5 (Vergleichsbeispiel)

Es wurde verfahren wie unter Beispiel 2. Das in das nach der Polymerisation erhaltene Polymergel eingearbeitete Unterkorn wurde vor der Rückführung mit Sipernat® D17 (Evonik Industries AG; Essen; DE) beschichtet. Sipernat® D17 ist eine pyrogene Kieselsäure.

Die erhaltenen wasserabsorbierenden Polymerpartikel wurden analysiert. Die Ergebnisse sind in Tabelle 1 zusammengefasst.

Herstellung des mit Sipernat® D17 beschichteten Unterkorns:

Dazu wurden 84 g Unterkorn und 0,25 g Sipernat® D17 in eine 250 ml Glasflasche eingefüllt und mit einem Rollenmischer 30 Minuten homogenisiert.

Beispiel 6 (Vergleichsbeispiel)

Es wurde verfahren wie unter Beispiel 2. Das in das nach der Polymerisation erhaltene Polymergel eingearbeitete Unterkorn wurde vor der Rückführung mit Tricalciumphosphat C 13-09 (Chemische Fabrik Budenheim KG; Budenheim; DE) beschichtet.

Die erhaltenen wasserabsorbierenden Polymerpartikel wurden analysiert. Die Ergebnisse sind in Tabelle 1 zusammengefasst.

Herstellung des mit Tricalciumphosphat C 13-09 beschichteten Unterkorns:

Dazu wurden 84 g Unterkorn und 0,42 g Tricalciumphosphat C 13-09 in eine 250 ml Glasflasche eingefüllt und mit einem Rollenmischer 30 Minuten homogenisiert.

Tabelle 1: Grundpolymere

| Bsp. Vgl. | Rückführung | CRC [g/g] | AUL0.3psi [g/g] | FSR [g/gs] | Remos [ppm] | CIE- Farbzahl | | |
|---|---|---|---|---|---|---|---|---|
| | | | | | | L* | b* | H60 |
| 1 | keine | 42,1 | 7,1 | 0,27 | 340 | 90,7 | 7,4 | 68,4 |
| 2 | unbeschichtetes Unterkorn | 39,8 | 7,3 | 0,31 | 509 | 91,1 | 7,9 | 67,5 |
| 3 | Unterkorn beschichtet mit Natriumhypophosphit | 40,6 | 6,9 | 0,30 | 821 | 91,1 | 6,5 | 71,7 |
| 4 | Unterkorn beschichtet mit Brüggolite® FF7 | 39,9 | 7,4 | 0,30 | 617 | 91,3 | 7,2 | 69,7 |
| 5 | Unterkorn beschichtet mit Sipernat® D17 | 40,2 | 7,2 | 0,28 | 313 | 91,7 | 7,8 | 68,4 |
| 6 | Unterkorn beschichtet mit Tricalciumphospat C 13-09 | 39,4 | 7,5 | 0,29 | 642 | 91,3 | 7,2 | 69,8 |

Beispiel 7 (Oberflächennachvemetzung) (Vergleichsbeispiel)

Das in Beispiel 1 hergestellte Grundpolymer wurde Oberflächennachvernetzt.

Dazu wurden 100 g Grundpolymer in eine Küchenmaschine eingefüllt und gerührt. Auf das gerührte Grundpolymer wurde innerhalb einer Minute mittels eines Zweistoffdüse 3,15 g einer wässrigen Lösung (0,15 g 2-Hydroxyethyl-2-oxazolidinon, 0,9 g Isopropanol und 2,1 g Wasser) aufgesprüht und 5 Minuten nachgerührt. Das beschichteten Grundpolymer wurde auf zwei Petrischalen (Durchmesser 20 cm; Höhe 2,5 cm) gleichmäßig verteilt und 60 Minuten bei 170°C im Umlufttrockenschrank oberflächennachvernetzt.

Nach der thermischen Oberflächennachvernetzung wurde über ein Sieb mit der Maschenweite von 850 $\mu$m Grobkorn abgetrennt, das Grobkorn quantitativ bestimmt und verworfen. Die so erhaltenen oberflächennachvernetzten wasserabsorbierenden Polymerpartikel mit einer Partikelgröße von 850 $\mu$m und weniger wurden analysiert. Die Ergebnisse sind in Tabelle 2 zusammengefasst.

Beispiel 8 (Vergleichsbeispiel)

Es wurde verfahren wie unter Beispiel 7. Eingesetzt wurde das in Beispiel 2 hergestellte Grundpolymer.

Die erhaltenen oberflächennachvernetzten wasserabsorbierenden Polymerpartikel wurden analysiert. Die Ergebnisse sind in Tabelle 2 zusammengefasst.

Beispiele 9 bis 12 (Vergleichsbeispiel)

Es wurde verfahren wie unter Beispiel 7. Eingesetzt wurden die in den Beispielen 3 bis 6 hergestellten Grundpolymere.

Die erhaltenen oberflächennachvernetzten wasserabsorbierenden Polymerpartikel wurden analysiert. Die Ergebnisse sind in Tabelle 2 zusammengefasst.

Tabelle 2: Oberflächennachvernetzung

| Bsp. Vgl. | Rückführung | CRC [g/g] | AUL0.7psi [g/g] | FSR [g/gs] | Remos [ppm] | Partikel >850$\mu$m [Gew.-%] | CIE-Farbzahl | | |
|---|---|---|---|---|---|---|---|---|---|
| | | | | | | | L | b | H60 |
| 7 | keine | 38,5 | 8,0 | 0,31 | 918 | 3,5 | 88,6 | 7,9 | 64,8 |
| 8 | unbeschichtetes Unterkorn | 35,3 | 8,5 | 0,30 | 1131 | 4,5 | 89,7 | 8,2 | 65,0 |

(fortgesetzt)

| Bsp. Vgl. | Rückführung | CRC [g/g] | AUL0.7psi [g/g] | FSR [g/gs] | Remos [ppm] | Partikel >850μm [Gew.-%] | CIE-Farbzahl | | |
|---|---|---|---|---|---|---|---|---|---|
| | | | | | | | L | b | H60 |
| 9 | Unterkorn beschichtet mit Natriumhypophosphit | 35,4 | 7,8 | 0,33 | 871 | 1,4 | 90,1 | 6,8 | 69,6 |
| 10 | Unterkorn beschichtet mit Brüggolite® FF7 | 36,5 | 7,7 | 0,35 | 638 | 0,5 | 90,0 | 7,6 | 67,1 |
| 11 | Unterkorn beschichtet mit Sipernat® D17 | 35,0 | 8,8 | 0,32 | 697 | 1,8 | 90,4 | 8,1 | 66,1 |
| 12 | Unterkorn beschichtet mit Tricalciumphospat C 13-09 | 35,4 | 8,5 | 0,32 | 776 | 2,1 | 90,5 | 7,7 | 67,3 |

Die Ergebnisse belegen, dass die gemäß dem erfindungsgemäßen Verfahren hergestellten oberflächennachvernetzte Polymerpartikel eine höhere Anquellgeschwindigkeit, weniger Restmonomere, weniger Agglomerate (Partikel >850 μm) und eine weißere Farbe mit geringem Gelbanteil (H60) aufweisen.

## Patentansprüche

1. Verfahren zur Herstellung wasserabsorbierender Polymerpartikel durch Polymerisation einer Monomerlösung oder -suspension, enthaltend

   a) mindestens ein ethylenisch ungesättigtes, säuregruppentragendes Monomer,
   b) mindestens einen Vernetzer,
   c) mindestens einen Initiator,
   d) optional ein oder mehrere mit den unter a) genannten Monomeren copolymerisierbare ethylenisch ungesättigte Monomere und
   e) optional ein oder mehrere wasserlösliche Polymere,

   umfassend die Schritte

   i) Polymerisation der Monomerlösung oder -suspension zu einem Polymergel,
   ii) Trocknung des Polymergels,
   iii) Zerkleinerung des getrockneten Polymergels und
   iv) Klassierung, wobei Unterkorn abgetrennt wird,

   wobei das abgetrennte Unterkorn zumindest teilweise vor Schritt ii) rückgeführt wird, **dadurch gekennzeichnet, dass** die wasserabsorbierenden Polymerpartikel vor Schritt iv) oberflächennachvernetzt werden, dass nach Schritt iii) und vor der Oberflächennachvernetzung zusätzlich klassiert und Unterkorn abgetrennt wird und dass zumindest eine Teilmenge des rückgeführten Unterkorns mit mindestens einem Reduktionsmittel und/oder anorganischen Partikeln beschichtet ist, wobei sie vor Schritt iv) und nach der Oberflächennachvernetzung mit einem Reduktionsmittel und/oder anorganischen Partikeln beschichtet wird.

2. Verfahren gemäß Anspruch 1, **dadurch gekennzeichnet, dass** das vor der Oberflächennachvernetzung abgetrennte Unterkorn zusammen mit dem in Schritt iv) abgetrennten Unterkorn rückgeführt wird.

3. Verfahren gemäß einem der Ansprüche 1 bis 2, **dadurch gekennzeichnet, dass** das abgetrennte Unterkorn nach Schritt i) rückgeführt wird.

4. Verfahren gemäß einem der Ansprüche 1 bis 3, **dadurch gekennzeichnet, dass** die Teilmenge des rückgeführten Unterkorns, die mit dem Reduktionsmittel und/oder anorganischen Partikeln beschichtet ist, von 1 bis 20 Gew.-% beträgt, bezogen auf den Feststoffgehalt der Monomerlösung oder -suspension.

**5.** Verfahren gemäß einem der Ansprüche 1 bis 4, **dadurch gekennzeichnet, dass** das mit einem Reduktionsmittel beschichtete Unterkorn mit 0,01 bis 5 Gew.-% Reduktionsmittel beschichtet ist, bezogen auf das Unterkorn.

**6.** Verfahren gemäß einem der Ansprüche 1 bis 5, **dadurch gekennzeichnet, dass** das Reduktionsmittel ein Salz der unterphosphorigen Säure oder ein Salz einer Sulfinsäure ist.

**7.** Verfahren gemäß einem der Ansprüche 1 bis 6, **dadurch gekennzeichnet, dass** das mit anorganischen Partikeln beschichtete Unterkorn mit 0,05 bis 2 Gew.-% anorganischen Partikeln beschichtet ist, bezogen auf das Unterkorn.

**8.** Verfahren gemäß einem der Ansprüche 1 bis 7, **dadurch gekennzeichnet, dass** die anorganischen Partikel pyrogene Kieselsäure oder wasserunlösliche Salze der Phosphorsäure sind.

**9.** Verfahren gemäß einem der Ansprüche 1 bis 8, **dadurch gekennzeichnet, dass** das Monomer a) teilweise neutralisierte Acrylsäure ist.

**10.** Verfahren gemäß einem der Ansprüche 1 bis 9, **dadurch gekennzeichnet, dass** die wasserabsorbierenden Polymerpartikel eine Zentrifugenretentionskapazität von mindestens 15 g/g aufweisen.

**Claims**

**1.** A process for producing water-absorbing polymer particles by polymerizing a monomer solution or suspension comprising

    a) at least one ethylenically unsaturated monomer bearing acid groups,
    b) at least one crosslinker,
    c) at least one initiator,
    d) optionally one or more ethylenically unsaturated monomers copolymerizable with the monomers specified under a) and
    e) optionally one or more water-soluble polymers, comprising the steps of

        i) polymerizing the monomer solution or suspension to give a polymer gel,
        ii) drying the polymer gel,
        iii) comminuting the dried polymer gel and
        iv) classifying to remove undersize,

the removed undersize being recycled at least partially before step ii), wherein the water-absorbing polymer particles are surface postcrosslinked before step iv), step iii) is followed and the surface postcrosslinking is preceded by additional classification and undersize removal, and at least a portion of the recycled undersize is coated with at least one reducing agent and/or inorganic particles, wherein it is coated with a reducing agent and/or inorganic particles before step iv) and after the surface postcrosslinking.

**2.** The process according to claim 1, wherein the undersize removed before the surface postcrosslinking is recycled together with the undersize removed in step iv).

**3.** The process according to claim 1 or 2, wherein the undersize removed is recycled after step i).

**4.** The process according to any one of claims 1 to 3, wherein the portion of the recycled undersize coated with the reducing agent and/or inorganic particles is from 1 to 20% by weight, based on the solids content of the monomer solution or suspension.

**5.** The process according to any one of claims 1 to 4, wherein the undersize coated with a reducing agent is coated with from 0.01 to 5% by weight of reducing agent, based on the undersize.

**6.** The process according to any one of claims 1 to 5, wherein the reducing agent is a salt of hypophosphorous acid or a salt of a sulfinic acid.

**7.** The process according to any one of claims 1 to 6, wherein the undersize coated with inorganic particles is coated with from 0.05 to 2% by weight of inorganic particles, based on the undersize.

8. The process according to any one of claims 1 to 7, wherein the inorganic particles are fumed silica or water-insoluble salts of phosphoric acid.

9. The process according to any one of claims 1 to 8, wherein the monomer a) is partially neutralized acrylic acid.

10. The process according to any one of claims 1 to 9, wherein the water-absorbing polymer particles have a centrifuge retention capacity of at least 15 g/g.

**Revendications**

1. Procédé de fabrication de particules polymères absorbant l'eau par polymérisation d'une solution ou suspension de monomères, contenant

    a) au moins un monomère éthyléniquement insaturé, portant des groupes acides,
    b) au moins un agent de réticulation,
    c) au moins un initiateur,
    d) éventuellement un ou plusieurs monomères éthyléniquement insaturés copolymérisables avec les monomères cités en a), et
    e) éventuellement un ou plusieurs polymères solubles dans l'eau,

comprenant les étapes suivantes :

    i) la polymérisation de la solution ou suspension de monomères pour former un gel polymère,
    ii) le séchage du gel polymère,
    iii) le broyage du gel polymère séché et
    iv) la classification, un tamisat étant séparé,

le tamisat séparé étant recyclé au moins en partie avant l'étape ii), **caractérisé en ce que** les particules polymères absorbant l'eau sont post-réticulées en surface avant l'étape iv), **en ce qu'**une classification et une séparation d'un tamisat sont également réalisées après l'étape iii) et avant la post-réticulation de surface et **en ce qu'**au moins une partie du tamisat recyclé est revêtue avec au moins un agent réducteur et/ou des particules inorganiques, dans lequel elle est revêtue avec un agent réducteur et/ou des particules inorganiques avant l'étape iv) et après la post-réticulation de surface.

2. Procédé selon la revendication 1, **caractérisé en ce que** le tamisat séparé avant la post-réticulation de surface est recyclé conjointement avec le tamisat séparé à l'étape iv).

3. Procédé selon l'une quelconque des revendications 1 et 2, **caractérisé en ce que** le tamisat séparé est recyclé après l'étape i).

4. Procédé selon l'une quelconque des revendications 1 à 3, **caractérisé en ce que** la partie du tamisat recyclé, qui est revêtue avec l'agent réducteur et/ou des particules inorganiques, est de 1 à 20 % en poids, par rapport à la teneur en solides de la solution ou suspension de monomères.

5. Procédé selon l'une quelconque des revendications 1 à 4, **caractérisé en ce que** le tamisat revêtu avec un agent réducteur est revêtu avec 0,01 à 5 % en poids d'agent réducteur, par rapport au tamisat.

6. Procédé selon l'une quelconque des revendications 1 à 5, **caractérisé en ce que** l'agent réducteur est un sel de l'acide hypophosphoreux ou un sel d'un acide sulfinique.

7. Procédé selon l'une quelconque des revendications 1 à 6, **caractérisé en ce que** le tamisat revêtu avec des particules inorganiques est revêtu avec 0,05 à 2 % en poids de particules inorganiques, par rapport au tamisat.

8. Procédé selon l'une quelconque des revendications 1 à 7, **caractérisé en ce que** les particules inorganiques sont de la silice pyrogénée ou des sels insolubles dans l'eau de l'acide phosphorique.

**9.** Procédé selon l'une quelconque des revendications 1 à 8, **caractérisé en ce que** le monomère a) est l'acide acrylique partiellement neutralisé.

**10.** Procédé selon l'une quelconque des revendications 1 à 9, **caractérisé en ce que** les particules polymères absorbant l'eau présentent une capacité de rétention centrifuge d'au moins 15 g/g.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0463388 A1
- EP 0496594 A2
- EP 0785224 A2
- EP 1878761 A1
- US 5064582 A
- WO 2004018006 A1
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 2002032962 A2
- WO 2001038402 A1
- DE 3825366 A1
- US 6241928 B
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 2003031482 A1
- DE 3713601 A1

**In der Beschreibung aufgeführte Nicht-Patentliteratur**

- **F.L. BUCHHOLZ ; A.T. GRAHAM.** Modern Superabsorbent Polymer Technology. Wiley-VCH, 1998, 71-103
- Ullmann's Encyclopedia of Industrial Chemistry. vol. 35, 73-93
- **HUNTERLAB.** *Band 8,* 1996, vol. 7